# EUROPEAN PATENT APPLICATION

(11) **EP 4 497 732 A1**
(43) Date of publication of application: **29.01.2025**
(21) Application number: 23761419.3
(22) Date of filing: 25.07.2023
(51) Int. Cl.: C03C 3/097

(54) **AMORPHOUS PHASE LITHIUM SILICATE GLASS PRODUCT, AND PREPARATION METHOD THEREFOR AND USE THEREOF**

(30) Priority: 14.06.2023 CN 202310698706
(71) Applicant: Aidite (Qinhuangdao) Technology Co., Ltd., Qinhuangdao Hebei 066004 (CN)
(72) Inventor: ZHAO, Lijia, Qinhuangdao, Hebei 066004 (CN); ZHANG, Jiaxin, Qinhuangdao, Hebei 066004 (CN); LI, Hongwen, Qinhuangdao, Hebei 066004 (CN); LIU, Manshu, Qinhuangdao, Hebei 066004 (CN); ZHANG, Nan, Qinhuangdao, Hebei 066004 (CN); XIONG, Xinyu, Qinhuangdao, Hebei 066004 (CN)
(74) Representative: Calysta NV
(86) International application number: PCT/CN2023/109036
(87) International publication number: WO 2024/254947

(57) **Abstract**

Provided are an amorphous lithium silicate glass product, and a preparation method and use thereof, which relate to the technical field of dental restoration materials. The method for preparing an amorphous lithium silicate glass product includes: subjecting a starting glass to heat treatment to obtain the amorphous lithium silicate glass product; wherein the starting glass is prepared from raw materials including SiO₂, Li₂O, Al₂O₃, P₂O₅, and an additive; and the heat treatment is conducted at a temperature of 350°C to 560°C. The amorphous lithium silicate glass product has neither adhesion in a microstructure nor cracks in a macroscopic view, and shows a desirable marginal stability and an excellent machinability. Meanwhile, the amorphous lithium silicate glass product has a relatively high linear translucency.

## Description

### CROSS REFERENCE TO RELATED APPLICATION

The application claims the priority and benefit of Chinese Patent Application No. 2023106987069, entitled "Amorphous lithium silicate glass product, and preparation method and use thereof" filed with the China National Intellectual Property Administration (CNIPA) on June 14, 2023, the disclosure of which is incorporated herein by reference in its entirety as part of the application.

### TECHNICAL FIELD

The present disclosure relates to the technical field of dental restoration materials, in particular to an amorphous lithium silicate glass product, and a preparation method and use thereof.

### BACKGROUND

Starting glasses obtained by melting glass-ceramic raw materials are in a glass state. During the milling and grinding of restoration preparation, pure glass materials are prone to cracking, resulting in poor marginal stability of the restoration. As a result, after wearing restoration, the marginal line does not fit tightly. The damages caused by a loose fitting of the marginal line mainly include accumulation of plaque, generation or aggravation of periodontal inflammations, and excessive bonding gap, which may make the adhesive too thick. Under the action of a long-term chewing pressure, the adhesive breaks and dissolves, resulting in secondary caries at the edge of the restoration.

At present, lithium disilicate glass-ceramics obtained by high-temperature crystallization (generally greater than 700°C) are generally used as dental restoration products clinically. For example, Chinese patent publication No. CN113501668A and No. CN106413626A both disclose that the materials containing lithium disilicate crystals are used as dental restoration materials. However, due to the microscopic rod-like interlocking structure of the lithium disilicate material, when milling directly, on the one hand, it is easy to cause damage to the milling bur, and on the other hand, it may also cause large cracks on the edge of the restoration, thereby affecting the final positioning of the restoration.

In order to solve the processing problem of materials, an intermediate state-lithium metasilicate state during the formation of lithium disilicate materials has been found by material manufacturers, and it is easier to be milled than that in the lithium disilicate state. Therefore, the easy-to-mill lithium metasilicate products currently dominate the market. Such products are milled and ground after one or more heat treatments (at not less than 600°C) to precipitate a lithium metasilicate ceramic phase inside the glass (the ceramic phase generally accounts for not less than 50%), which can ensure the grinding and milling effect to a certain extent. EP2284133B1 discloses that lithium silicate products with lithium metasilicate as a main crystal phase are used for the manufacture of a dental restoration. Such dental restoration has an extremely high proportion of the ceramic phase inside, leading to greater wear on the milling bur; moreover, during milling or grinding lamellar or spherical lithium metasilicate grains, it is unavoidable that microstructure adhesion could cause macrostructure cracks, resulting in poor marginal stability.

Moreover, traditional lithium disilicate glass-ceramic materials and lithium metasilicate products have low translucency, making it impossible to observe defects in dental restoration during processing/preparation. Accordingly, when patients wear restoration and perform daily activities such as chewing, the defects on the restoration may soon be exposed. This is generally manifested as fracture of the restoration clinically.

### SUMMARY

An object of the present disclosure is to provide an amorphous lithium silicate glass product, and a preparation method and use thereof. In the present disclosure, the amorphous lithium silicate glass product has neither adhesion in a microstructure nor cracks in a macroscopic view, and shows a desirable marginal stability and an excellent machinability. Meanwhile, the amorphous lithium silicate glass product has a relatively high linear translucency.

To achieve the above object of the present disclosure, the following technical solutions are provided.

The present disclosure provides a method for preparing an amorphous lithium silicate glass product, including:
subjecting a starting glass to heat treatment to obtain the amorphous lithium silicate glass product;
wherein the starting glass is prepared from raw materials including SiO₂, Li₂O, Al₂O₃, P₂O₅, and an additive; and
the heat treatment is conducted at a temperature of 350°C to 560°C.

In some embodiments, the starting glass is prepared by a process including: subjecting the raw materials of the starting glass to first melting to obtain a glass melt, and pouring the glass melt into water to obtain a frit; and subjecting the frit to second melting to obtain a glass liquid, and subjecting the glass liquid to pouring moulding to obtain the starting glass.

In some embodiments, the first melting is conducted at a temperature of 1,250°C to 1,600°C for 1 h to 8 h.

In some embodiments, the second melting is conducted at a temperature of 1,400°C to 1,600°C for 1 h to 24 h.

In some embodiments, the pouring moulding is performed by a process including pouring, cooling and solidification, and annealing in sequence; and
the annealing is conducted at a temperature of 200°C to 300°C for 1 h to 20 h.

In some embodiments, the heat treatment is conducted for 2 min to 20 h.

In some embodiments, the heat treatment is performed once (i.e., one heat treatment) or multiple times (i.e., multiple heat treatments); and the multiple heat treatments is conducted by a process including: subjecting the starting glass to first heat treatment by heating to a heat treatment temperature, and then cooling to room temperature with furnace temperature, and repeating the first heat treatment and the cooling with furnace temperature; alternatively, the multiple heat treatments is conducted by subjecting the starting glass to a gradient heat treatment.

The present disclosure further provides an amorphous lithium silicate glass product prepared by the method as described in the above solutions.

In some embodiments, the amorphous lithium silicate glass product has a linear translucency of 75% to 95%.

The present disclosure further provides use of the amorphous lithium silicate glass product as described in the above solutions in preparation of a dental restoration material.

The present disclosure provides a method for preparing an amorphous lithium silicate glass product. In the present disclosure, a starting glass is subjected to heat treatment at a temperature of 350°C to 560°C so that a large number of small crystal nuclei are precipitated inside the amorphous lithium silicate glass product. Due to a small size of the crystal nuclei, the product is still in a state of glass macroscopically, and the microscopic components cannot be detected by X-ray diffraction (XRD). These crystal nuclei have a large number and are independent of each other, and there is no adhesion in the microstructure. As a result, there are no cracks macroscopically after milling, and the marginal stability is better. Since the small nuclei material is easier to be milled, more material can be milled with a same bur than the ceramic phase. The amorphous lithium silicate glass product according to the present disclosure is more bur-saving and has excellent machinability. The results of the examples show that when a same set of burs is adopted, only 30 to 50 pieces of lithium silicate products with lithium metasilicate as the main crystal phase could be milled, while not less than 80 pieces of amorphous lithium silicate glass products of the present disclosure could be milled.

Furthermore, the amorphous lithium silicate glass product of the present disclosure has a relatively high linear translucency, preferably 75% to 95%. In the present disclosure, the amorphous lithium silicate glass product with high linear translucency could be used to prepare dental restoration materials, so that during processing/preparation, the transparent state makes it easier to detect defects caused by the processing of the restoration. In this way, on the one hand, a rework rate of the restoration can be reduced; and on the other hand, a fracture risk of the restoration after the patient wears restoration can also be reduced.

In addition, lithium metasilicate state glass-ceramic products have high heat treatment temperature and long treatment time, leading to high energy consumption. In the present disclosure, the heat treatment shows relatively low temperature and relatively short treatment time, thereby resulting in more energy saving.

### BRIEF DESCRIPTION OF THE DRAWINGS

FIG. 1 shows an XRD pattern of the amorphous lithium silicate glass product prepared in Example 1.
FIG. 2 shows an XRD pattern of a lithium silicate product prepared in Comparative Example 1.
FIG. 3 shows a process flow diagram of a gradient heat treatment in Example 7.
FIG. 4 shows an XRD pattern of the amorphous lithium silicate glass products prepared in Examples 2 to 7.

### DETAILED DESCRIPTION OF THE EMBODIMENTS

The present disclosure provides a method for preparing an amorphous lithium silicate glass product, which includes the following steps:
subjecting a starting glass to heat treatment to obtain the amorphous lithium silicate glass product;
wherein the starting glass is prepared from raw materials including SiO₂, Li₂O, Al₂O₃, P₂O₅, and an additive; and
the heat treatment is conducted at a temperature of 350°C to 560°C.

In some embodiments, a starting glass is subjected to heat treatment to obtain the amorphous lithium silicate glass product. In some embodiments, the starting glass is prepared by a process including: subjecting the raw materials of the starting glass to first melting to obtain a glass melt, and pouring the glass melt into water to obtain a frit; and subjecting the frit to second melting to obtain a glass liquid, and subjecting the glass liquid to pouring moulding to obtain the starting glass.

In some embodiments, the raw materials of the starting glass are mixed in the same container, and the mixing is manual mixing or mechanical mixing. In some embodiments, the first melting is conducted at a temperature of 1,250°C to 1,600°C, preferably 1,400°C to 1,550°C, and more preferably 1,450°C to 1,500°C. In some embodiments, the first melting is conducted for 1 h to 8 h, preferably 2 h to 5 h, and more preferably 3 h to 4 h. In some embodiments, the first melting is conducted in air. In the present disclosure, the glass melt obtained after the first melting is poured into water, so as to be directly quenched into the frit. In some embodiments, the second melting is conducted at a temperature of 1,400°C to 1,600°C, preferably 1,450°C to 1,580°C, and more preferably 1,500°C to 1,550°C. In some embodiments, the second melting is conducted for 1 h to 24 h, preferably 8 h to 24 h, and more preferably 10 h to 20 h. In some embodiments, the second melting is conducted in air. The second melting makes the composition distribution of the starting glass more uniform.

In some embodiments, the pouring moulding is performed by a process including pouring, cooling and solidification, and annealing in sequence. In some embodiments, the pouring is conducted in a mould. There is no special requirement on a shape of the mould, which could be determined according to the needs of the product. In some embodiments, the cooling and solidification is conducted at room temperature. In some embodiments, the annealing is conducted at a temperature of 200°C to 300°C, preferably 240°C to 280°C, and more preferably 250°C to 260°C. In some embodiments, the annealing is conducted for 1 h to 20 h, preferably 2 h to 15 h, and more preferably 8 h to 12 h. In some embodiments, the annealing is conducted in air.

In some embodiments, the starting glass is prepared from the raw materials including 55 wt% to 75 wt% of the SiO₂, 10 wt% to 25 wt% of the Li₂O, 0.5 wt% to 12 wt% of the Al₂O₃, 2 wt% to 8 wt% of the P₂O₅, 0.1 wt% to 11 wt% of the additive, and 0 wt% to 10 wt% of a colorant.

In some embodiments, the raw materials of the starting glass include 55 wt% to 75 wt%, preferably 58 wt% to 73 wt%, and more preferably 59 wt% to 65 wt% of the SiOz.

In some embodiments, the raw materials of the starting glass include 10 wt% to 25 wt%, preferably 13 wt% to 22 wt%, and more preferably 15 wt% to 20 wt% of the LiO₂.

In some embodiments, the raw materials of the starting glass include 0.5 wt% to 12 wt%, preferably 1 wt% to 8 wt%, and more preferably 2 wt% to 6 wt% of the Al₂O₃.

In some embodiments, the raw materials of the starting glass include 2 wt% to 8 wt%, preferably 4 wt% to 6 wt%, and more preferably 5 wt% of the P₂O₅.

In some embodiments, the raw materials of the starting glass include 0.1 wt% to 11 wt%, preferably 2.3 wt% to 10.8 wt%, and more preferably 6.1 wt% to 8.3 wt% of the additive. In some embodiments, the additive includes one or more selected from the group consisting of an alkali metal oxide, a divalent metal oxide, a trivalent metal oxide, and a tetravalent metal oxide. In some embodiments, the alkali metal oxide includes one or more selected from the group consisting of Na₂O and K₂O. In some embodiments, the divalent metal oxide includes one or more selected from the group consisting of BeO, MgO, CaO, SrO, BaO, and ZnO. In some embodiments, the trivalent metal oxide includes one or more selected from the group consisting of B₂O₃, La₂O₃, and Bi₂O₃. In some embodiments, the tetravalent metal oxide includes one or more selected from the group consisting of ZrO₂ and TiOz. The additive is intended to adjust an addition amount and a proportion of each component, so as to adjust a viscosity of the glass system, a crystallization time, and a crystal growth direction during the crystallization.

In some embodiments, the additive includes one or more selected from the group consisting of Na₂O, K₂O, MgO, BaO, ZnO, B₂O₃, ZrO₂, and TiO₂. In a specific embodiment, a mass ration of Na₂O, K₂O, MgO, BaO, ZnO, B₂O₃, ZrO₂, and TiO₂ is in a range of (0-1):(0-0.2):(0-1):(0-0.3):(0-3):(0-2):(1-8):(0-1), preferably (0.1-0.5):0.2:(0.1-0.5):(0.2-0.3):(1-2.4):(0.1-2):(2-5):(0.1-0.5).

In some embodiments, the raw materials of the starting glass further include 0 wt % to 10 wt %, preferably 2.7 wt % to 6.9 wt %, and more preferably 3.1 wt % to 4.7 wt % of the colorant. In some embodiments, the colorant includes one or more selected from the group consisting of a manganese-containing compound, a cobalt-containing compound, a nickel-containing compound, a copper-containing compound, a chromium-containing compound, a vanadium-containing compound, an iron-containing compound, and a rare earth oxide. In some embodiments, the manganese-containing compound is MnO₂; the cobalt-containing compound is CoO; the nickel-containing compound is Ni₂O₃; the copper-containing compound is CuO; the chromium-containing compound is Cr2O3; the vanadium-containing compound is V₂O₅; the iron-containing compound is Fe₂O₃; and the rare earth oxide includes one or more selected from the group consisting of Er₂O₃, CeO₂, and Nd₂O₃.

In some embodiments, the colorant includes one or more selected from the group consisting of CoO, Fe₂O₃, Er₂O₃, CeO₂, and Nd₂O₃. In a specific embodiments, a mass ration of CoO, Fe₂O₃, Er₂O₃, CeO₂, and Nd₂O₃ is in a range of(0-0.1):(0-0.2):(0-2):(2-4):(0-0.8), preferably (0-0.1):(0.1-0.2):(0.1-0.5):(2.5-3.5):(0.1-0.5), and more preferably 0.1:(0.1-0.2):(0.2-0.3):(3.0-3.5):(0.2-0.4).

After the starting glass is obtained, the starting glass is subj ected to heat treatment to obtain the amorphous lithium silicate glass product. In some embodiments, the heat treatment is conducted at a temperature of 350°C to 560°C, preferably 400°C to 555°C, and more preferably 420°C to 500°C. In some embodiments, the heat treatment is conducted for 2 min to 20 h, preferably 20 min to 15 h, and more preferably 3 h to 5 h. In some embodiments, A temperature of the heat treatment is obtained by heating at a rate of 5°C/h to 200°C/h, preferably 20°C/h to 50°C/h from room temperature.

In some embodiments, the heat treatment is performed once (i.e. one heat treatment) or multiple times (i.e. multiple heat treatments). In some embodiments, the multiple heat treatments is conducted 1 to 5 times, preferably 1 to 3 times.

In some embodiments, the multiple heat treatments is conducted by a process including: subjecting the starting glass to first heat treatment by heating to a heat treatment temperature, cooling to room temperature with furnace temperature, and repeating the first heat treatment and the cooling with furnace temperature. In some embodiments, during the multiple heat treatments, each heat treatment is conducted at a temperature of 350°C to 560°C independently, preferably 400°C to 555°C independently, and more preferably 420°C to 500°C independently. In some embodiments, each heat treatment is conducted for 2 min to 20 h independently, preferably 20 min to 15 h independently, and more preferably 3 h to 5 h independently.

In some embodiments, the multiple heat treatments is conducted by subjecting the starting glass to a gradient heat treatment. In some embodiments, the gradient heat treatment is conducted by a process including: heating to an intermediate temperature at a first heating rate and conducting first heat preservation, and heating to a final temperature at a second heating rate and conducting second heat preservation. In some embodiments, the first heating rate is in a range of 50°C/h to 200°C/h and preferably 100°C/h to 150°C/h. In some embodiments, the intermediate temperature is in a range of 350°C to 450°C and preferably 380°C to 420°C. In some embodiments, the first heat preservation is conducted for 3 h to 20 h and preferably 5 h to 15 h. In some embodiments, the second heating rate is in a range of 5°C/h to 30°C/h and preferably 10°C/h to 20°C/h. In some embodiments, the second heat preservation is conducted for 20 min to 20 h and preferably 1 h to 10 h. In some embodiments, the final temperature is in a range of 450°C to 560°C and preferably 500°C to 555°C. In a specific embodiment, the gradient heat treatment is conducted by heating at a rate of 50°C/h to 420°C and holding for 3 h, and then heating at a rate of 30°C/h to 555°C and holding for 20 min.

In the present disclosure, a product obtained by heat treatment with the above parameters is in a glass state or a glass-like state, and is the amorphous lithium silicate glass product. In some preferred embodiments, the multiple heat treatments is used as heat treatment, which are more conducive to improving the machinability of the amorphous lithium silicate glass product.

In some embodiments, the heat treatment is conducted in air. In some embodiments, after the heat treatment is completed, a resulting product is cooled to room temperature with furnace temperature to obtain the amorphous lithium silicate glass product.

The present disclosure further provides an amorphous lithium silicate glass product prepared by the method as described in the above solutions. In some embodiments, the amorphous lithium silicate glass product has a linear translucency of 75% to 95%, preferably 80% to 90%.

The present disclosure further provides use of the amorphous lithium silicate glass product in preparation of a dental restoration material, preferably in preparation of a dental restoration. In some embodiments, the dental restoration includes one or more selected from the group consisting of a veneer, an inlay, a full crown, and a bridge. There is no special requirement on a specific method of the use, and the use methods well-known to those skilled in the art may be used.

The technical solutions of the present disclosure will be clearly and completely described below with reference to the examples of the present disclosure. Apparently, the described examples are merely a part rather than all of the embodiments of the present disclosure. All other embodiments obtained by those skilled in the art based on the embodiments of the present disclosure without creative efforts shall fall within the scope of the present disclosure.

### Examples

The raw materials of a starting glass were mixed in the same container; a resulting fully mixed raw materials were subjected to first melting, and a resulting glass melt was directly poured into water to be quenched into a frit.

The frit was subjected to second melting, a resulting glass liquid was poured into a mould, and then cooled, solidified, and annealed in sequence to obtain the starting glass.

The starting glass was subjected to heat treatment and cooled to room temperature with furnace temperature to obtain an amorphous lithium silicate glass product.

In these examples, the raw materials and process parameters for preparing the starting glass are shown in Table 1.

Table 1 Raw material compositions and process parameters used in examples

| | Component | Example 1 | Example 2 | Example 3 | Example 4 | Example 5 | Example 6 |
|---|---|---|---|---|---|---|---|
| Raw material composition of starting glass (wt%) | SiO₂ | 65 | 59 | 60 | 73 | 63 | 58 |
| | Li₂O | 15 | 20 | 17 | 10 | 13 | 22 |
| | Al₂O₃ | 5 | 8 | 6 | 2 | 3 | 1 |
| | P₂O₅ | 5 | 8 | 6 | 2 | 8 | 4 |
| | Na₂O | 1 | 0.1 | 0.5 | 0.3 | 0.1 | / |
| | K₂O | / | / | 0.2 | / | / | 0.2 |
| | MgO | 1 | / | 0.1 | / | 0.5 | 0.2 |
| | BaO | / | 0.2 | / | / | / | 0.3 |
| | ZnO | 2 | / | 3 | 1 | 2.4 | 2 |
| | B₃O₃ | / | / | 0.1 | 2 | / | / |
| | Z₁O₂ | 2 | 1 | 3 | 5 | 3 | 8 |
| | TiO₂ | 0.5 | 1 | 1 | / | 0.1 | 0.1 |
| | CoO | / | / | 0.1 | / | 0.1 | / |
| | Fe₂O₃ | / | 0.1 | 0.1 | 0.1 | / | 0.2 |
| | Er₂O₃ | / | 0.2 | 0.3 | 0.1 | 2 | 0.5 |
| | CeO₂ | 3.5 | 2 | 2.5 | 4 | 4 | 3 |
| | Nd₃O₃ | / | 0.4 | 0.1 | 0.5 | 0.8 | 0.5 |
| First melting | Temperature | 1500°C | 1550°C | 1550°C | 1600°C | 1450°C | 1500°C |
| | Time | 3h | 2h | 5h | 3h | 8h | 5h |
| Second melting | Temperature | 1550°C | 1580°C | 1580°C | 1580°C | 1600°C | 1500°C |
| | Time | 3h | 2h | 6h | 10h | 2h | 20h |
| Annealing | Temperature | 300°C | 200°C | 280°C | 260°C | 300°C | 240°C |
| | Time | 2h | 10h | 8h | 10h | 12h | 15h |
| Heat treatment | Temperature | 555°C | 560°C | 350°C | 560°C | 420°C | 500°C |
| | Time | 20min | 3h | 20h | 5h | 20h | 15h |

### Example 7

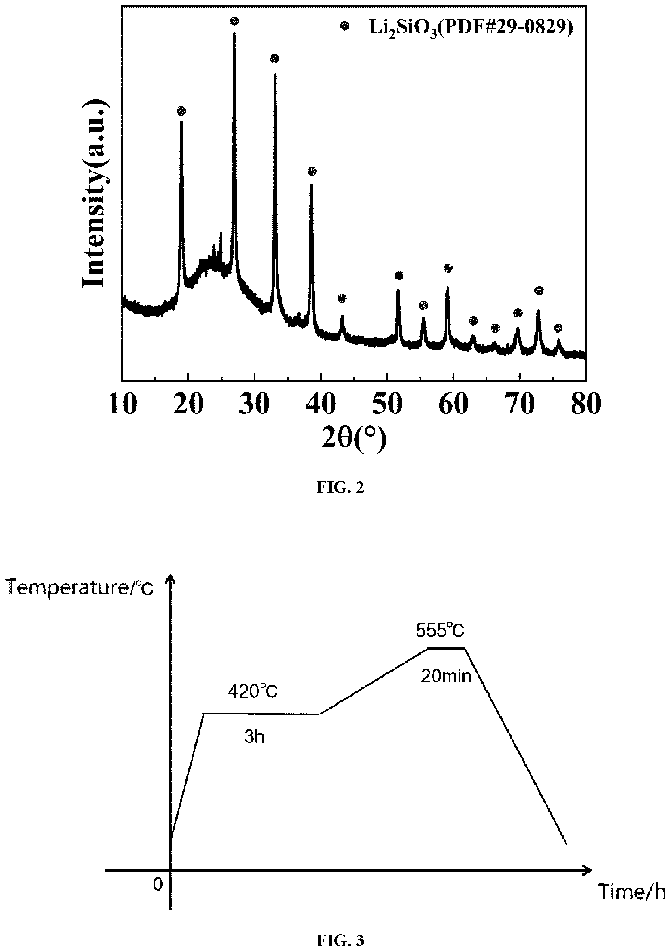

This example was performed as described in Example 1, except that the heat treatment was a gradient heat treatment. As shown in FIG. 3, the specific process was performed as follows: the starting glass was heated to an intermediate temperature of 420°C at a rate of 50°C/h and held for 3 h, and then heated to a final temperature of 555°C at a rate of 30°C/h and held for 20 min; and a resulting product was cooled to room temperature with furnace temperature to obtain an amorphous lithium silicate glass product.

### Comparative Example 1

This comparative example was performed as described in Example 1, except that the heat treatment was conducted at 650°C, and a resulting sample was a lithium silicate product with lithium metasilicate as a main crystal phase.

### Comparative Example 2

This comparative example was performed as described in Example 1, except that the heat treatment was conducted at 300°C to obtain a glass block.

### Test Example 1

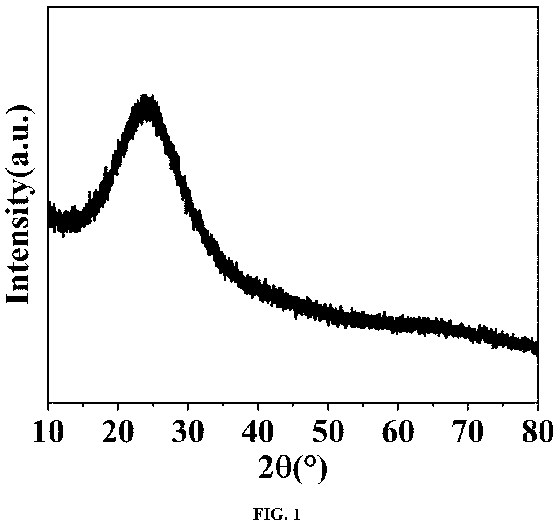

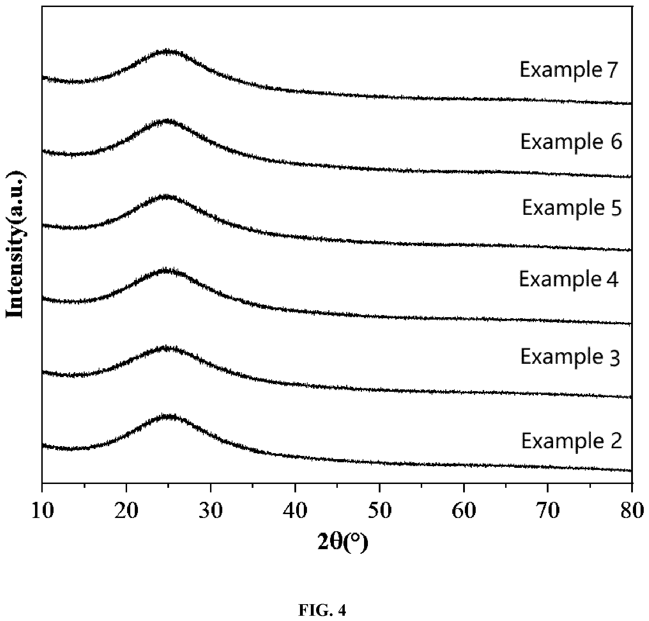

FIG. 1 shows an XRD pattern of the amorphous lithium silicate glass product prepared in Example 1. As shown in FIG. 1, the sample prepared in Example 1 has a "steamed bread-shaped peak" in XRD, indicating that it is a typical amorphous sample. FIG. 4 shows an XRD pattern of the amorphous lithium silicate glass products prepared in Examples 2 to 7. Similar to that shown in FIG. 1, indicating that these samples are typical amorphous samples.

FIG. 2 shows an XRD pattern of the lithium silicate product prepared in Comparative Example 1. As shown in FIG. 2, due to a higher heat treatment temperature in Comparative Example 1, the resulting sample is a lithium silicate product with lithium metasilicate as a main crystal phase.

The product prepared in Comparative Example 2 is a glass block, and during milling of such glass block, the dental restoration will be broken and could not be completely milled.

### Test Example 2

The samples prepared in Examples 1 to 7 and Comparative Example 1 were milled using 8 sets of identical brand-new burs, respectively. The amorphous lithium silicate glass product prepared in Example 1 could be milled into 80 dental restoration; the amorphous lithium silicate glass product prepared in Example 2 could be milled into 88 dental restoration; the amorphous lithium silicate glass product prepared in Example 3 could be milled into 83 dental restoration; the amorphous lithium silicate glass product prepared in Example 4 could be milled into 84 dental restoration; the amorphous lithium silicate glass product prepared in Example 5 could be milled into 82 dental restoration; the amorphous lithium silicate glass product prepared in Example 6 could be milled into 85 dental restoration; the amorphous lithium silicate glass product prepared in Example 7 could be milled into 100 dental restoration; and the lithium silicate product prepared in Comparative Example 1 could be milled into 32 dental restoration, and then the bur could no longer be used. In view of the above, it could be concluded that the amorphous lithium silicate glass product prepared according to the present disclosure is easier to be milled and saved on burs.

### Test Example 3

The samples prepared in Examples 1 to 7 and Comparative Examples 1 to 2 were separately sawn into small pieces of length × width × thickness = 25 mm × 25 mm × 1 mm, and a linear translucency of each piece corresponding to different sample was tested using a haze meter. The results are shown in Table 2.

Table 2 Linear translucency of samples prepared in Examples 1 to 7 and Comparative Examples 1 to 2

| Sample | Linear translucency (Tt), % |
|---|---|
| Example 1 | 90.63 |
| Example 2 | 91.72 |
| Example 3 | 85.65 |
| Example 4 | 84.95 |
| Example 5 | 88.23 |
| Example 6 | 89.65 |
| Example 7 | 84.56 |
| Comparative Example 1 | 25.75 |
| Comparative Example 2 | 90.12 |

As shown in Table 2, the amorphous lithium silicate glass products prepared according to the present disclosure have a higher linear translucency. During processing/preparation of dental restoration materials, the transparent state makes it easier to detect defects of the restoration. In this way, on the one hand, a rework rate of the restoration could be reduced; and on the other hand, a fracture risk of the restoration after the patient wears the restoration could also be reduced.

The above descriptions are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

1. A method for preparing an amorphous lithium silicate glass product, comprising:
subjecting a starting glass to heat treatment to obtain the amorphous lithium silicate glass product;
wherein the starting glass is prepared from raw materials comprising SiO₂, Li₂O, Al₂O₃, P₂O₅, and an additive; and
the heat treatment is conducted at a temperature of 350°C to 560°C.

2. The method of claim 1, wherein the starting glass is prepared by a process comprising:
subjecting the raw materials of the starting glass to first melting to obtain a glass melt, and pouring the glass melt into water to obtain a frit; and
subjecting the frit to second melting to obtain a glass liquid, and subjecting the glass liquid to pouring moulding to obtain the starting glass.

3. The method of claim 2, wherein the first melting is conducted at a temperature of 1,250°C to 1,600°C for 1 h to 8 h.

4. The method of claim 2, wherein the second melting is conducted at a temperature of 1,400°C to 1,600°C for 1 h to 24 h.

5. The method of claim 2, wherein the pouring moulding is performed by a process comprising pouring, cooling and solidification, and annealing in sequence; and
the annealing is conducted at a temperature of 200°C to 300°C for 1 h to 20 h.

6. The method of claim 1, wherein the starting glass is prepared from the raw materials comprising 55 wt% to 75 wt% of the SiO₂, 10 wt% to 25 wt% of the Li₂O, 0.5 wt% to 12 wt% of the Al₂O₃, 2 wt% to 8 wt% of the P₂O₅, 0.1 wt% to 11 wt% of the additive, and 0 wt% to 10 wt% of a colorant.

7. The method of claim 6, wherein the additive comprises one or more selected from the group consisting of an alkali metal oxide, a divalent metal oxide, a trivalent metal oxide, and a tetravalent metal oxide.

8. The method of claim 6 or 7, wherein the additive comprises one or more selected from the group consisting of Na₂O, K₂O, MgO, BaO, ZnO, B₂O₃, ZrOz, and TiO₂.

9. The method of claim 1, wherein the heat treatment is conducted for 2 min to 20 h.

10. The method of claim 1, wherein the heat treatment is performed once (i.e., one heat treatment) or multiple times (i.e., multiple heat treatments); and
the multiple heat treatments is conducted by a process comprising:
subjecting the starting glass to first heat treatment by heating to a heat treatment temperature, and then cooling to room temperature with furnace temperature, and
repeating the first heat treatment and the cooling with furnace temperature;
alternatively, the multiple heat treatments is conducted by subjecting the starting glass to a gradient heat treatment.

11. The method of claim 10, wherein the gradient heat treatment is conducted by a process comprising:
heating to an intermediate temperature at a first heating rate and conducting first heat preservation, and
heating to a final temperature at a second heating rate and conducting second heat preservation;
the first heating rate is in a range of 50°C/h to 200°C/h, the intermediate temperature is in a range of 350°C to 450°C, and the first heat preservation is conducted for 3 h to 20 h; and
the second heating rate is in a range of 5°C/h to 30°C/h, the final temperature is in a range of 450°C to 560°C, and the second heat preservation is conducted for 20 min to 20 h.

12. An amorphous lithium silicate glass product prepared by the method of any one of claims 1 to 11.

13. The amorphous lithium silicate glass product of claim 12, wherein the amorphous lithium silicate glass product has a linear translucency of 75% to 95%.

14. Use of the amorphous lithium silicate glass product of any one of claims 12 to 13 in preparation of a dental restoration material.
